# EUROPEAN PATENT APPLICATION

(11) **EP 3 210 642 A1**
(43) Date of publication of application: **30.08.2017**
(21) Application number: 14904479.4
(22) Date of filing: 22.10.2014
(51) Int. Cl.: A61M 16/00, A62B 7/00

(54) **BREATHING BAG**

(71) Applicant: Shenzhen Yuantai Medical Equipment Co., Ltd., Shenzhen, Guangdong 518000 (CN)
(72) Inventor: HUANG, Jun rong, Shenzhen Guangdong 518000 (CN)
(74) Representative: Jeannet, Olivier
(86) International application number: PCT/CN2014/089129
(87) International publication number: WO 2016/061749

(57) **Abstract**

Disclosed is a breathing bag, comprising three components, A, B and C. The weight ratio of the three components, A, B and C is 100: 90 to 400: 1 to 15, wherein the component A comprises PVC, the component B comprises a plasticizer, and the component C comprises a stabilizer. The Component A is a powder, the component B is a liquid, and the component C is a liquid or a powder. The breathing bag is mainly made of PVC, and a certain amount of a plasticizer and a stabilizer are added, so that the breathing bag does not contain natural rubber latexes to which a patient may be allergic; and the production process does not require the use of talcum powder and ammonia, thus reducing the pollution of the environment.

## Description

### BACKGROUND

### 1. Technical Field

The invention relates to a breathing bag, particularly in related to a breathing bag made of PVC (Polyvinylchloride, PVC).

### 2. Description of Related Art

The current breathing bags are generally made from the natural latex, but some patients are allergic to the natural latex, which will cause discomfort or even be fatal to patients in the course of using.

Just because breathing bags made of natural latex are with these shortcomings, there have been some breathing bags made from other materials, such as synthetic Latex. However, talcum powder preventing adhesion to breathing bags as well as ammonia used to raise the PH are required in the process of synthetic Latex breathing bags production, which will cause the pollution to the environment. Therefore, how to overcome the above shortcomings of existing breathing bags is an urgent problem needed to be solve.

### SUMMARY

The purpose of the present invention is to provide a breathing bag made of PVC.

In order to solve the above problems, the present invention provides the following technology programs.

A breathing bag is provided, which includes a component A, a component B and a component C. The weight ratio of the component A, the component B and the component C is 100: 90 to 400: 1 to 15. The component A includes PVC, the component B includes a plasticizer, and the component C includes a stabilizer. The component A is a powder, the component B is a liquid, and the component C is a liquid or a powder.

Wherein the PVC includes a PVC fine powder and a PVC meal powder. The weight ratio of the PVC fine powder and the PVC meal powder is 100:1 to 50.

Wherein the PVC includes a PVC fine powder.

Wherein the stabilizer includes a heat stabilizer and a fixer. The weight ratio of the heat stabilizer and the fixer is 1:1.

Wherein the stabilizer includes a heat stabilizer or a fixer.

Wherein the breathing bag further includes a component D, and the component D includes a tear-resistant agent.

Wherein the weight ratio of the components A, B, C, and D is 100:90 to 400:1 to 15:1 to 40.

Wherein the breathing bag further includes a component E, and the E component includes an elasticizer.

Wherein the weight ratio of the components A, B, C, D, and E is 100:90 to 200:1 to 15:1 to 20.

Wherein the breathing bag further includes a component F, and the F component includes a coupling agent.

Wherein the weight ratio of the components A, B, C, D, E and F is 100:90 to 200:1 to 15:1 to 20:1 to 6.

Due to the breathing bag of the present invention being mainly made from PVC and added with a certain amount of plasticizer and stabilizer, the breathing bag of the present invention does not contain natural latex which is allergic to patients, and does not need talcum powder and ammonia in the production process. Therefore, environmental pollution is reduced.

### DETAILED DESCRIPTION

The breathing bag provided according to the first embodiment of the present invention, includes a component A, a component B and a component C. The weight ratio of the component A, the component B and the component C is 100: 90 to 400: 1 to 15. The component A includes PVC (Polyvinylchloride), the component B includes a plasticizer, and the component C includes a stabilizer. The component A is a powder, the component B is a liquid, and the component C is a liquid or a powder.

According to one selectable embodiment of the present invention, the PVC includes the PVC fine powders and the PVC meal powders. The PVC fine powders are produced by emulsion or micro-suspension method with an average grain diameter of 5∼30 µm or less. The PVC fine powders are usually tied with the additives into a paste, formed by means of knife coating, impregnation as well as slushing, and then is heated and plastically molded to be the products. The PVC meal powders are produced by bulk polymerization or suspension method with an average grain diameter of 50∼200 µm.

Furthermore, the weight ratio of the PVC fine powder and the PVC meal powder is 100:1 to 50. Specifically, the PVC fine powder is the KM-31 produced in Korea or the CPM-31 produced in China. The PVC meal powder is the SLP-1000 produced in China. It should be noticed that the above PVC model is only for example. The PVC of the present invention does not rule out other common PVC in the industry.

In other embodiments, PVC can also comprise the PVC fine powder only, and does not comprise the PVC meal powder.

Because the PVC meal powders are added to the PVC fine powder and PVC and the particles of PVC meal powders are larger than the PVC fine powder particles, the surface roughness of the breathing bag is increasingly made, which further prevents the adhesion between the breathing bag. Meanwhile, since the made breathing bags are no adhesion to each other, the talcum powders used to separate the breathing bags in the production process are not necessary. Therefore, the environmental pollution is reduced and the environmental pollution problem due to the necessity of using the talcum powders to separate the breath bags is also solved.

In one selectable embodiment of the present invention, the plasticizers are the environmentally friendly plasticizers, such as Acetyl Tributyl Citrate (ATBC). It should be noticed that the above plasticizers is only for example. The plasticizers of the present invention do not rule out other common plasticizers in the industry.

In one selectable embodiment of the present invention, the stabilizer includes a heat stabilizer and a fixer. The weight ratio of the heat stabilizer and the fixer is 1:1.

In other embodiments, the stabilizer includes a heat stabilizer or a fixer, which means the stabilizer only need comprising one of heat stabilizer and a fixer.

The heat stabilizer is R39 heat stabilizer produced in United States, which is used to enhance the stability of breathing bags to light as well as heat and prolong the service life of the breathing bag. Of course, the heat stabilizers of the present invention of can also be the other thermal stabilizers, such as D39 heat stabilizers, Ca/Zn composite stabilizer and epoxy soybean oil. Meanwhile, it should be noticed that the above stabilizers are only for example. The stabilizers of the present invention do not rule out other common stabilizers in the industry.

The stabilizer, such as CZ-118, is used to enable the mixtures used in manufacturing breathing bags to be placed for a long time without deterioration, which improves the quality of the breathing bags. Of course, the stabilizers of the present invention can also be the other common stabilizers in the industry.

The breathing bags provided according to the second embodiment of the present invention, further includes the component D in addition to the component A, the component B and the component C in the first embodiment, the component D includes a tear-resistant agent. Wherein the weight ratio of the components A, B, C, and D is 100:90 to 400:1 to 15:1 to 40.

Specifically, the tear resistance agents in the present invention, such as silica, is used to improve the tear resistance of breathing bags, which prevents the breathing bag be teared accidentally. Of course, the tear resistance agents of the present invention can also be the other common tear resistance agents in the industry, such as the HR-580 tear resistance agent produced in the United States.

In this embodiment, the component D is powder. In other embodiments, the component D is liquid.

The breathing bags provided according to the third embodiment of the present invention, further includes the component E in addition to the components A, B, C, and D in the first embodiment. The component D includes a elasticizer. Wherein the weight ratio of the components A, B, C, D, and E is 100:90 to 200:1 to 15:1 to 40:1 to 20.

Specifically, the elasticizer in the present invention, such as liquid nitrile rubber (LNBR), is used to reduce the brittleness of the breathing bags and increase the impact resistance and flexibility of breathing bags. Of course, the elasticizer of the present invention can also be the other common elasticizer in the industry, such as the 26NEP-VR54 elasticizer produced in the United States.

In this embodiment, the component E is liquid. In other embodiments, the component E is powder.

The breathing bags provided according to the fourth embodiment of the present invention, further includes the component F in addition to the components A, B, C, D, and E in the third embodiment. The component F includes a coupling agent. Wherein the weight ratio of the components A, B, C, D, E and F is 100:90 to 200:1 to 15:1 to 40:1 to 20:1 to 6.

In particular, the coupling agent in the present invention, such as the silane coupling agent kh570, is used to improve the affinity between the various components of breathing bags and increase the correlation between various components. Of course, the coupling agent of the present invention can also be the other common coupling agent in the industry. Specifically, when the components D and E is the type of powder, the component F is needed to be added.

In this invention, the stabilizers, the tear-resistant agent, the elasticizer and the coupling agent are additives.

Because the main component of the breathing bag in the invention is PVC which is nontoxic and renewable resources, and the used elasticizer and additives are environmentally friendly, the allergic reaction does not occur in the patient who use the breathing bag made of PVC in the invention. Also talcum powder and aqueous ammonia are not required in the production process, which further reduces the environmental pollution and solves environmental pollution problems caused by using Latex or synthetic Latex as raw material to manufacture the breathing bag.

Meanwhile, elasticizers, stabilizers and other additives are added in PVC, which makes the breathing bag of the present invention having good elasticity as well as thermal stability and increases the service life of the breathing bag.

Further, the tear-resistant agent is also added in PVC, which further improves the tear resistance of the breathing bag.

Further, the elasticizers is also added in PVC, which further reduces the brittleness of the breathing bag, increases the impact resistance of the breathing bag, and improves the elasticity of the breathing bag.

Further, the coupling agent is also added in PVC, which further improves the wear resistance of the breathing bag.

In this embodiment, the above different components are evenly mixed to form the mixture, and then breath bags are manufactured by means of dipping.

Described above are the preferred embodiments of the invention. It should be noted that, for the skilled persons in this technical field, some improvements and refinements may also be made on the premise of not from principles of the invention. The improvements and refinements should also be considered as the protection scope of the present invention.

## Claims

1. A breathing bag, comprising a component A, a component B and a component C having the weight ratio of 100: 90 to 400: 1 to 15, wherein the component A comprises PVC, the component B comprises a plasticizer, and the component C comprises a stabilizer, wherein the component A is a powder, the component B is a liquid, and the component C is one of a liquid and a powder.

2. The breathing bag as claimed in claim 1, wherein the PVC comprises a PVC fine powder and a PVC meal powder, wherein the weight ratio of the PVC fine powder and the PVC meal powder is 100:1 to 50.

3. The breathing bag as claimed in claim 1, wherein the PVC comprises a PVC fine powder.

4. The breathing bag claimed in any one of claims 1 to 3, wherein the stabilizer comprises a heat stabilizer and a fixer, the weight ratio of the heat stabilizer and the fixer is 1:1.

5. The breathing bag as claimed in any one of claims 1 to 3, wherein the stabilizer comprises a heat stabilizer or a fixer.

6. The breathing bag as claimed in any one of claims 1 to 5, wherein the breathing bag further comprises a component D, and the D component comprises a tear-resistant agent.

7. The breathing bag as claimed in claim 6, wherein the weight ratio of the components A, B, C, and D is 100:90 to 400:1 to 15:1 to 40.

8. The breathing bag as claimed in claim 6, wherein the breathing bag further comprises a component E, and the E component comprises an elasticizer.

9. The breathing bag as claimed in claim 8, **characterized in that** the weight ratio of the components A, B, C, D and E is 100:90 to 200:1 to 15:1 to 40:1 to 20.

10. The breathing bag as claimed in claim 8, wherein the breathing bag further comprises a component F, and the F component comprises a coupling agent.

11. The breathing bag as claimed in claim 10, wherein the weight ratio of the components A, B, C, D, E and F is 100:90 to 200:1 to 15:1 to 40:1 to 20:1 to 6.
